# EUROPEAN PATENT APPLICATION

(11) **EP 4 840 546 A1**
(43) Date of publication of application: **18.11.2026**
(21) Application number: 25175965.0
(22) Date of filing: 13.05.2025
(51) Int. Cl.: B01L 9/00

(54) **APPARATUS AND METHOD FOR GRIPPING A DISPENSER, AND A LIQUID HANDLING APPARATUS**

(71) Applicant: CurifyLabs Oy, 00180 Helsinki (FI)
(72) Inventor: BARINOFF, Mikko, 00180 Helsinki (FI); SIIMES, Aslak, 00180 Helsinki (FI); NGUYEN, Tri, 00180 Helsinki (FI); JANTUNEN, Hannes, 00180 Helsinki (FI); SANDLER, Niklas, 00180 Helsinki (FI)
(74) Representative: Laine IP Oy

(57) **Abstract**

According to an example aspect of the present invention, there is provided an apparatus for gripping a dispenser, the apparatus comprising a main body comprising a cam disc, a bottom plate, and at least one jaw assembly; a split nut; a split nut body adjacent to the main body, said split nut body configured to house the split nut; and a handle assembly fixedly attached to the main body, each jaw assembly comprising a jaw slider, a spacer, and a jaw.

## Description

### FIELD

This disclosure provides a solution for gripping dispensers, specifically for apparatuses in the field of liquid handling applications. More specifically, the present disclosure provides a liquid handling apparatus comprising an apparatus for gripping a dispenser, and a method of gripping the dispenser using said apparatus.

### BACKGROUND

In laboratories or similar scientific or industrial settings, it is beneficial for liquid handling apparatuses to be robust, precise, and easy to use. In such settings, liquid handling systems are commonly used for routine or highly precise dispensing tasks. To ensure accuracy and efficiency, dispensers such as syringes or cartridges must be securely mounted to the apparatuses. Proper attachment mechanisms are essential to facilitate controlled movement and reliable dispensing.

The present disclosure provides an apparatus and method for gripping a dispenser. More specifically, the present disclosure provides an apparatus and a method for easy attachment and detachment of such dispensers to the apparatuses, using a self-centering attachment mechanism.

### SUMMARY OF THE INVENTION

The invention is defined by the features of the independent claims. Some specific embodiments are defined in the dependent claims.

According to a first aspect of the present invention, there is provided an apparatus for gripping a dispenser, the apparatus comprising a main body comprising a cam disc, a bottom plate, and at least one jaw assembly; a split nut; a split nut body adjacent to the main body, said split nut body configured to house the split nut; and a handle assembly fixedly attached to the main body, each jaw assembly comprising a jaw slider, a spacer, and a jaw.

Some embodiments of the first aspect may comprise at least one feature from the following bulleted list:
- wherein the apparatus is configured to grip a dispenser, for example a movable part of the dispenser, in particular a plunger flange of a syringe, wherein the main body is configured to move the at least one jaw assembly away from the center of the cam disc upon application of force to the cam disc, for example where the force is applied to the cam disc via the handle assembly,
- wherein the gripping comprises fixing the dispenser between the spacer and a tilted surface of the jaw,
- wherein the main body comprises at least two jaw assemblies; wherein the apparatus has a closed position, wherein the at least two jaw assemblies are arranged to contact each other; and an open position, wherein the at least two jaw assemblies define a space between them,
- wherein the main body is configured to disengage the split nut from a leadscrew when a force is applied to the handle assembly,
- wherein the apparatus is configured to simultaneously release the dispenser and disengage the split nut from the leadscrew when the force is applied to the handle assembly,
- wherein the main body comprises at least three jaw assemblies,
- wherein the main body further comprises a top plate,
- wherein the handle assembly comprises a moveable handle and a stationary handle, wherein the moveable handle is fixedly attached to the cam disc, and wherein the stationary handle is fixedly attached to the bottom plate or to the top plate,
- wherein the force comprises an input force conveyed in a suitable direction, such as a horizontally-directed input force, to the moveable handle of the handle assembly,
- wherein the apparatus is configured to be connected to a linear actuator via the leadscrew,
- wherein the underside of the cam disc comprises arched grooves configured to facilitate rotational movement of the cam disc, wherein the rotational movement of the cam disc causes the cam to come into contact with and actuate the split nut away from the leadscrew, thereby disengaging the split nut from the leadscrew,
- wherein the bottom plate comprises at least one groove configured to allow centripetal movement of at least one respective jaw slider,
- wherein the apparatus further comprises a housing configured to protect the apparatus,
- wherein the at least one jaw is configured to move centripetally away from the center of the cam disc, thereby releasing the dispenser, upon application of force to the handle assembly,
- wherein the at least one jaw comprises a tilted surface, wherein the tilted surface is configured to interlock with the dispenser, wherein the dispenser is interlocked between the upper edge of the tilted surface of the jaw and the bottom surface of the spacer,
- wherein the cam disc comprises at least one protrusion, wherein the at least one protrusion extends through an opening of the bottom plate
- wherein the apparatus further comprises a spring arranged around the center axle of the cam disc, wherein one end of the spring is fixedly attached within a spring housing of the cam disc, and another end is positioned adjacent to the outer edge of the cam disc

According to a second aspect of the present invention, there is provided a method of gripping a dispenser, the method comprising: applying a force to a movable handle of an apparatus, the apparatus configured so that at least one jaw assembly moves centripetally away from the center of a cam disc upon application of said force, wherein each jaw assembly comprises a jaw, a spacer, and a slider; placing a top surface of the dispenser against the underside of each spacer within an area defined by the at least one jaw assembly; and releasing the force on the movable handle, the apparatus configured so that the releasing of the force moves the at least one jaw assembly centripetally towards the center of the cam disc, thereby fixing the dispenser in a cavity within the at least one jaw assembly, whereby horizontal and vertical movement of the dispenser is prevented.

Some embodiments of the second aspect may comprise at least one feature from the following bulleted list:
- removing the dispenser from the apparatus by applying a force to the moveable handle after the releasing of the force
- applying an input force to the moveable handle, wherein said input force is applied by a user or by an automated device
- moving the apparatus along a vertical axis via a leadscrew
- wherein the applying of a force simultaneously disengages a split nut from a leadscrew, thereby allowing movement along a vertical axis via the leadscrew
- wherein the releasing of a force simultaneously engages the split nut to the leadscrew, thereby securing the apparatus to the leadscrew and preventing movement along a vertical axis

According to a third aspect of the present invention, there is provided a liquid handling apparatus, the liquid handling apparatus comprising at least the apparatus of the first aspect, and an actuator configured to actuate the apparatus of the first aspect along a vertical axis via a leadscrew.

Some embodiments of the third aspect may comprise at least one feature from the following bulleted list:
- wherein the liquid handling apparatus is configured to move along a horizontal axis in both x- and y-directions within an x-y coordinate system
- further comprising a device operatively connected to the liquid handling apparatus, wherein the device comprises at least one processing core, at least one memory including computer program code, the at least one memory and the computer program code being configured to, with the at least one processing core, cause the device to control movement of the liquid handling apparatus
- further comprising a screen, wherein the screen comprises a user interface configured to at least participate in controlling the movement of the liquid handling apparatus

### BRIEF DESCRIPTION OF THE DRAWINGS

FIGURE 1 illustrates an isometric view of an example of a gripping apparatus in accordance with at least some embodiments of the present disclosure.
FIGURE 2 illustrates an exemplary embodiment of a syringe in accordance with at least some embodiments of the present disclosure.
FIGURES 3A and 3B illustrate an isometric view of an example apparatus capable of supporting at least some embodiments of the present disclosure.
FIGURE 4A and 4B illustrate a bottom view of an example of a gripping apparatus in accordance with at least some embodiments of the present disclosure.
FIGURES 5A, 5B, and 5C illustrate an example of a cam disc in accordance with at least some embodiments of the present disclosure.
FIGURE 6 illustrates an example of a plurality of jaw assemblies in accord with at least some embodiments of the present disclosure from an isometric top view.
FIGURE 7 illustrates an example of a plurality of jaw assemblies in accord with at least some embodiments of the present disclosure from an isometric bottom view.
FIGURE 8 illustrates a top view of an exemplary embodiment of a cam disc and split nut in accord with at least some embodiments of the present disclosure.
FIGURES 9A and 9B illustrate an isometric view and a top view, respectively, of an exemplary embodiment of a split nut in accord with at least some embodiments of the present disclosure.
FIGURE 10 illustrates a split nut and a bottom plate in accord with at least some embodiments of the present disclosure from an isometric top view.
FIGURE 11 illustrates a bottom view of an example of a gripping apparatus in accordance with at least some embodiments of the present disclosure.
FIGURE 12 illustrates a bottom view of an example of a gripping apparatus in accordance with at least some embodiments of the present disclosure.
FIGURE 13 illustrates a liquid handling apparatus in accord with at least some embodiment of the present disclosure from an isometric top view.

### EMBODIMENTS

The present disclosure provides an apparatus for gripping a dispenser, for example a syringe or a cartridge, and a method of gripping the dispenser using said apparatus. Further, a liquid handling apparatus comprising the apparatus for gripping a dispenser is disclosed.

The apparatus and method are useful for dispensing operations, for example liquid or viscous fluid dispensing operations, in particular liquid or viscous fluid dispensing operations which use a syringe or a cartridge. For example, formulations which can be admixed with active pharmaceutical ingredients (API) to produce dispensable dispersions can be dispensed by the apparatus and/or method. By using the apparatus and/or method, the dispenser may be actuated in an accurate manner to provide precise dispensing. Further, the dispenser is attached to the apparatus in a resilient manner, without requiring extra manual clamps or screws. Further, the apparatus and methods herein are suitable for 3D printing, in particular of pharmaceutical products. 3D printing refers to a manufacturing process that creates three-dimensional objects, for example by successively adding material layer by layer to fabricate items, in particular items with precise geometries and complex structures.

### DEFINITIONS

A dispenser, for example a syringe, refers to a device used to draw in, contain, and dispense liquids, viscous fluids or other materials in various applications. A dispenser may be used to dispense for example liquids, semi-solids, viscous fluids, or formulations such as a base formulation, an excipient base, a drug formulation, or a medical formulation, which can be admixed with active pharmaceutical ingredients (API) to produce dispensable dispersions in precise volumes for applications such as laboratory procedures, industrial processes, or medical applications. A dispenser may be of different sizes depending on the intended use of the dispenser and the material to be dispensed. A cartridge is another example of dispenser that can be used for controlled dispensing of various formulations. Such a cartridge is typically pre-filled and designed for use in a dispensing system, ensuring precise and sterile delivery of for example liquids, semi-solids, viscous fluids, or formulations in pharmaceutical, industrial, and laboratory applications.

For example, a dispenser, such as a syringe or a cartridge, may have a volume capacity from 0.001 ml to 1000 ml, for example from 0.1 ml to 500 ml, or from 1 ml to 100 ml, or from 1 to 50 ml, or from 5 ml to 20 ml.

A syringe may comprise a barrel, a barrel flange, a plunger, a plunger flange, a seal, a luer lock, and a luer slip with a centric tip.

A syringe barrel may comprise a cylindrical main body for the material, for example liquid, which is to be dispensed. The barrel further may comprise barrel flanges located on the barrel. The barrel flanges are "wings" that protrude from the sides of the barrel and provide a surface or area which aids in aspiration or liquid administration by providing a surface to hold when the syringe is operated.

A syringe plunger is a shaft that connects the plunger flange to the syringe seal. The pressure applied to the plunger goes through to the seal and presses the liquid down towards the luer lock.

A plunger flange is a protruding feature at the rear end of the barrel that provides a gripping surface and easier application of pressure. The plunger flange bears the load of the pressure applied to the plunger.

A syringe seal is a flexible component positioned at the distal end of the plunger that forms a fluid-tight interface with the barrel's inner surface, preventing leakage and ensuring efficient fluid transfer. The seal slides up and down the barrel in response to the pressure applied to the plunger (similar to the motion of a piston in a cylinder).

A luer lock is a standardized screw fitting for attaching fittings to the syringe that will not 'blow off' when the liquid is under pressure. The luer lock ensures a leak-resistant interface. There are three different styles of luer lock; luer lock tip, luer slip centric tip, and luer slip eccentric tip. A syringe as used in the present disclosure may have any type of luer lock, preferably a luer slip centric tip. A luer slip centric tip is a luer lock with the tip centrally located. Reference is made to the ISO 80369 standard, for example part 7, with respect to the luer lock and luer slip.

Gripping, as used in this disclosure, means that a dispenser, for example a syringe or a cartridge, becomes fixed in an interlocked position between jaws of an apparatus, or between at least one jaw and at least one counterpart assembly. A counterpart assembly refers to a stationary counterpart to a jaw, configured to form an area where the dispenser can become fixed in an interlocked position together with at least one jaw. The apparatus may comprise a plurality of jaw assemblies, each jaw assembly comprising a jaw. When the dispenser is gripped, movement of the dispenser both vertically and horizontally is minimized or fully prevented. A plunger flange of a syringe may be gripped. Gripping occurs when the jaw assemblies move from an open position towards a closed position, for example, when an input force to a moveable handle of an apparatus is released. When gripped, the dispenser, for example a plunger flange of a syringe, is fixed in a cavity between the spacer and a tilted surface of each jaw, whereby horizontal and vertical movement of the dispenser is prevented.

Releasing (of a dispenser, for example a syringe), as used in this disclosure, means that a dispenser is freed from the interlocked position between the jaws in the plurality of jaw assemblies, or from the interlocked position between at least one jaw and at least one counterpart assembly, when the jaw assemblies move from a closed position to an open position. The jaw assemblies move from the closed position to the open position when an input force to the mechanism, for example via the movable handle, is applied. The input force may be a force that actuates the mechanism i.e., a force in any direction that causes the cam to move rotationally and the jaws to move centripetally. Preferably, the input force is a horizontally-directed input force to the moveable handle, for example from a user or automated device performing a pinching motion with a thumb and a finger.

The term horizontal, when used in this description, refers to a horizontal plane extending through the main body. The term vertical, when used in this description, refers to a vertical plane, perpendicular to the horizontal plane, extending through the main body. A horizontal direction may be parallel with the horizontal plane, which may be generally aligned with the ground, while a vertical direction may be parallel with the vertical plane, which may be generally aligned with the force of gravity.

The term centripetal or centripetally, when used in this description, refers to an outward or inward radial direction, for example, a direction from the center of a circle towards the edge of the circle, or a direction from the edge or near the edge of a circle towards the center, respectively. "Centripetally away" may therefore refer to an outward radial direction from the center of a circle. "Centripetally towards" may therefore refer to an inward radial direction from the edge or from any other part of the circle towards the centre point.

In this disclosure, several elements, parts, or assemblies are attached to each other. Such attachment may be facilitated by any of the following solutions singly or jointly: adhesive, hook-or-loop fastener, screws, bolts, clasps, locks, nails, snap-fits, pins, snap-fasteners, spring elements such as ball plungers, zip-ties, magnets, electro-magnets.

In this disclosure, parts may be actuated manually or automatically. Manual movement may be performed directly by the hand of an operator. Manual or automatic movement may also be performed using equipment comprising: a pushbutton, a handle, a lever, a pedal, a control apparatus, a hydraulic cylinder, a leadscrew, a bearing, a magnet, an electro-magnet, an actuator, an electric motor, a pneumatic cylinder, a linear variable differential transformer.

A liquid handling apparatus refers to an apparatus which is configured to dispense, extrude, or 3D print liquids, semi-solids, viscous fluids, or formulations which can be admixed with active pharmaceutical ingredients (API) to produce dispensable dispersions, by using a dispenser, for example a syringe. In such an apparatus, at least one motor is used to actuate the dispenser. Beneficially, such an apparatus further allows for moving the dispenser in at least two dimensions (X and Y dimensions), in particular in three dimensions (X, Y and Z) dimensions. This allows the apparatus to dispense to multiple locations, for example to one or more cavities of a container, to one or more blisters of a blister pack, or to a flat surface, for example a glass surface.

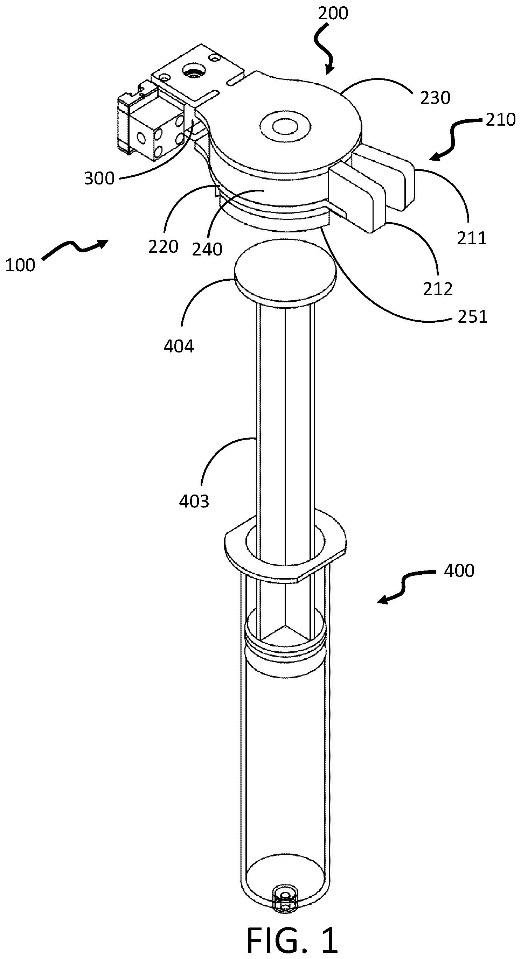

FIGURE 1 illustrates an exemplary embodiment of an apparatus in accordance with at least some embodiments of the present disclosure. In figure 1, a gripping apparatus 100 is illustrated, along with a syringe 400 suitable for gripping. Plunger 403 may be inserted, for example by a user, into the gripping apparatus. Specifically, the plunger flange 404 of the syringe may be inserted into the gripping apparatus 100 from underneath the gripping apparatus.

Figure 1 further shows a main body 200 and a split nut body 300 of the gripper 100. Main body 200 comprises a handle assembly 210, which further comprises a moveable handle 211 and a stationary handle 212, a bottom plate 220, a top plate 230, and a cam disc 240. The split nut body is configured to house a split nut. Additionally, an outer surface of a jaw 251 is shown.

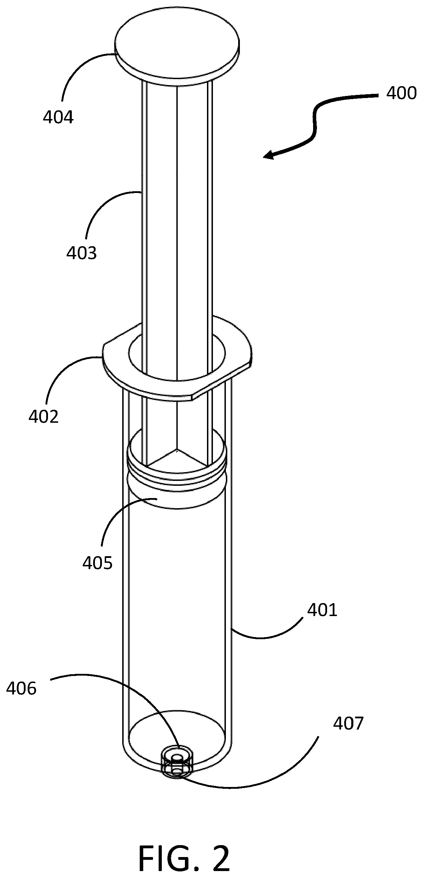

FIGURE 2 illustrates an exemplary embodiment of a syringe in accordance with at least some embodiments of the present disclosure. The syringe 400 comprises a barrel 401, a barrel flange 402, a plunger 403, a plunger flange 404, a seal 405, a luer lock 406, and a luer slip 407 with a centric tip. The syringe is shown as being transparent, so that the inner components are visible.

The barrel 401 comprises a cylindrical main body for containing the liquid which is to be dispensed. The barrel further comprises barrel flanges 402 located on the barrel. The barrel flanges are "wings" that protrude from the sides of the barrel and provide a surface or area which aids in aspiration or liquid administration.

The plunger 403 is a shaft that connects the plunger flange 404 to the seal 405. The pressure applied to the plunger 403 goes through to the seal 405 and presses the liquid down towards the luer lock 406.

The plunger flange 404 is a protruding feature at the rear end of the barrel 401 that provides a gripping surface and easier application of pressure. The plunger flange 404 bears the load of the pressure applied to the plunger 403.

The seal 405 is a flexible component positioned at the distal end of the plunger that forms a fluid-tight interface with the barrel's inner surface, preventing leakage and ensuring efficient fluid transfer. The seal slides up and down the barrel 401 in response to the pressure applied to the plunger 403. In other words, the seal is moved by the plunger towards the syringe tip, or away from the syringe tip, depending on the plunger's direction of movement.

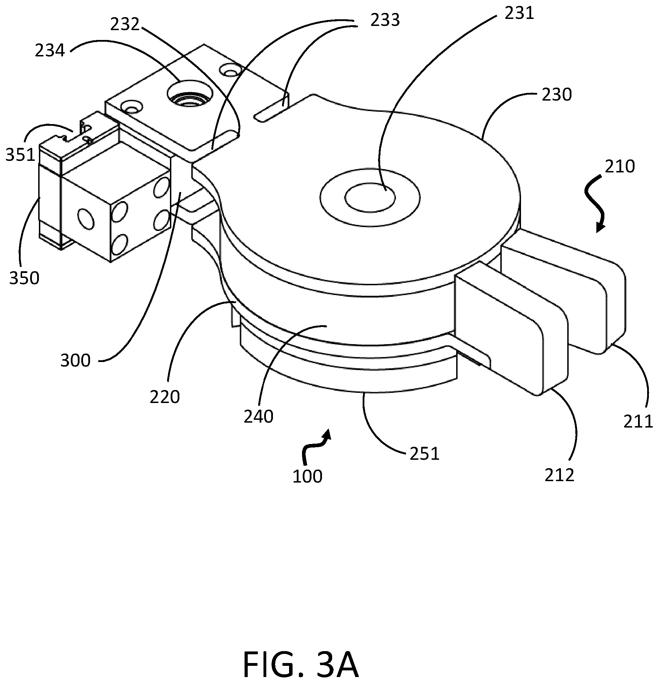

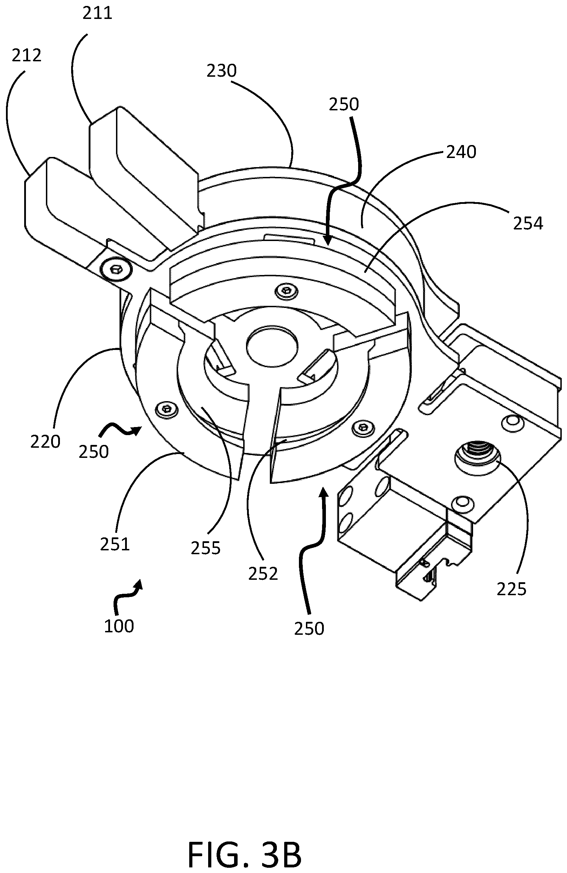

FIGURES 3A and 3B illustrate an example apparatus capable of supporting at least some embodiments of the present disclosure. In Figure 3A, gripping apparatus 100 is shown from an isometric top view. Gripping apparatus 100 is usable to grip a dispenser, for example syringe 400. The gripping apparatus 100 for gripping a syringe comprises a main body 200 and a split nut body 300. The main body 200 comprises a cam disc 240, a top plate 230, a bottom plate 220, and a handle assembly 210 comprising a moveable handle 211 and a stationary handle 212. Additionally, there is illustrated a linear guide block 350, which comprises a linear guide block groove 351. The linear guide block 350 is positioned adjacent to the split nut body 300. The linear guide block groove 351 is designed to facilitate smooth movement of the gripping apparatus 100 in the vertical direction along a guide rail with minimal friction when engaged with the rail.

The top plate rests upon the cam disc, and protects the cam disc from above. The top plate 230 further comprises a centrally-located top plate hole 231, a top plate bridge 232 which separates the main body from the split nut body, and gaps 233 on both sides of the bridge 232 which allow for an optional protective housing to be placed in the gaps 233. This top plate configuration provides mechanical support for the apparatus as a whole. Additionally, the top plate comprises an upper split nut hole 301. Split nut hole 301 is configured to allow a leadscrew to protrude through said split nut hole 301. The bottom plate 220 comprises a lower split nut hole 225. Said hole is arranged below (in the vertical direction) the split nut hole of the top plate. The leadscrew, when inserted into hole 234 or 225, will protrude through both holes 234 and 225. Thereby, the apparatus 100 may be moved up and down (in the vertical direction) by disengaging the split nut from the leadscrew.

The handle assembly comprises a moveable handle 211 and a stationary handle 212. The moveable handle may be fixedly attached to or an integral part of the cam disc 240. The movable handle may be configured to move towards the stationary handle along the periphery of the cam disc upon application of an input force to the mechanism, for example to the moveable handle of the handle assembly. In relation to the cam disc, the moveable handle may be configured to remain stationary, while being configured to move in relation to the stationary handle. The stationary handle may be fixedly attached to or an integral part of the bottom plate 220 or of the top plate 230. The stationary handle may be configured to remain stationary with respect to the bottom plate and/or top plate upon application of an input force to the mechanism, for example to the moveable handle of the handle assembly. For example, a user may place their thumb on the movable handle, and their index finger on the stationary handle, or the other way around, and actuate the movable handle using a pinching motion. The configuration of the handles as seen in Figure 3A provides good ergonomics for the user.

Figure 3B illustrates the main body from an isometric bottom view. The main body 200 comprises the cam disc 240, the top plate 230, the bottom plate 220, the handle assembly comprising the moveable handle 211 and the stationary handle 212, and three jaw assemblies 250. Each jaw assembly 250 in the plurality of jaw assemblies comprises a jaw 251, a jaw slider 253, and a spacer 254. Further, the bottom surface 255 of the spacers, and the inner sides 252 of the jaws 251, are illustrated. When a dispenser is placed in the apparatus, it is the top surface of the dispenser that is placed against the bottom surfaces 255 of the spacers, while an input force is applied to acuate the mechanism, for example via the moveable handle, and the jaws have moved centripetally away from the center axis for a distance long enough to allow the dispenser to fit in between the inner sides 252 of the jaws 251. The top surface of a dispenser is, for example, the top surface of a plunger flange 404 of a syringe 400.

The top plate 230 may comprise a centrally-located hole for the cam disc to protrude through. In addition, the top plate may extend over the split nut body, and rest upon the split nut body. In such embodiments, the top plate comprises a bridge between the main body and the split nut body. A gap may be formed on each side of the bridge, which allows for an optional housing 530. The optional housing is a protective housing of for example the split nut body, the split nut, the linear guide block, and the linear guide rail.

The bottom plate 220 provides a base for the cam disc. The bottom plate comprises a plurality of bottom plate grooves (holes), which allow the jaw assemblies to move in a centripetal direction. A bottom plate groove may be in the shape of a rectangle. Preferably, the apparatus comprises the same amount of bottom plate grooves and jaw assemblies. In a preferred embodiment, the apparatus comprises three jaw assemblies, whereby the apparatus is configured to grip a syringe with a three-point attachment. In addition, the bottom plate may optionally extend to the split nut body, and provide a base for the split nut body. In such embodiments, the bottom plate may comprise a bridge 223 between the main body and the split nut body. A gap may be formed on each side of the bridge 223, similar to the gaps in the top plate. The gaps in the top plate may be positioned above the gaps in the bottom plate and thereby be vertically aligned with each other.

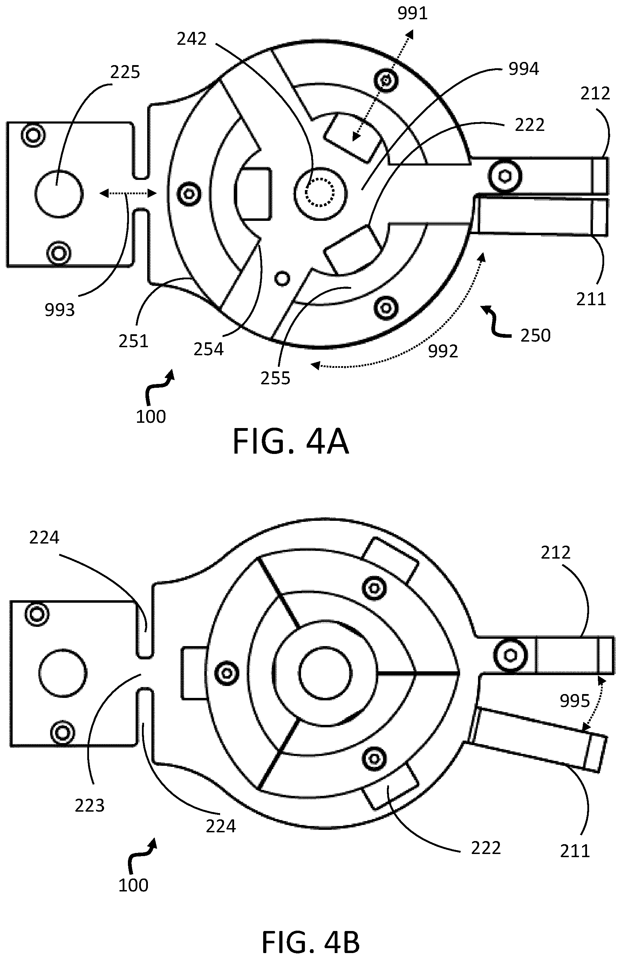

FIGURE 4A illustrates an exemplary embodiment of a gripping apparatus 100 in the open position from below. In the open position, the gripping apparatus is configured so that each jaw assembly in the plurality of jaw assemblies is located at a maximum distance from the centrally-located hole in the bottom plate. The maximum distance is defined by the length of the bottom plate grooves which only allow a certain amount of movement for the jaw sliders. In the open position, the jaw sliders are positioned at the outer ends of the bottom plate grooves. In the open position, there may be spaces or gaps 257 between each jaw assembly. In the open position, there may be spaces or gaps between at least one jaw assembly and the at least one counterpart assembly. In the open position, the volume, indicated by the reference number 994, between the jaw assemblies may be of a suitable size to allow a user to manually or automatically place a dispenser or a component thereof against the bottom surface of the spacer in each jaw assembly, and release the input force to allow the jaw assemblies to move towards the closed position to grip the syringe. In other words, the jaw assemblies are configured so that, when the gripping apparatus is in the open position, the dispenser will fit into the cavity of the gripper which is defined by the outer edges of surfaces 255 in the horizontal plane, and by the surfaces 255 themselves in the vertical plane. The volume of a suitable size means that the top surface of a dispenser, for example the top surface of a plunger flange of a syringe, may easily be placed towards the bottom surface of the spacer in each jaw assembly, without contacting the bottom protrusion of the cam disc or the underside of the bottom plate.

Figure 4A further illustrates three jaw assemblies 250. The jaws 251 and the spacers 254 are positioned below the rectangular bottom plate grooves 222. The jaw sliders 253 are positioned above the rectangular bottom plate grooves 222. The primary movement direction of the jaw assemblies is indicated by arrow 991 on one of the jaw assemblies. Additionally, the bottom surfaces 255 of the spacers 254 are depicted. In figure 4A, the jaw assemblies are shown at the outer endpoint of the bottom plate grooves 222, meaning the gripping apparatus is fully open. In this figure, an input force is applied to the mechanism, in this embodiment via the movable handle 211, enough to bring it into contact with the stationary handle 212. The handles are seen on the right side of the figure. When an input force is applied and subsequently released, the jaws automatically move towards the closed position. For example, the mechanism may be tensioned by a spring so that the jaws are moved to the closed position when an external force to the mechanism is applied and subsequently released. The split nut hole 225 of the bottom plate is visible from below. During operation, a leadscrew may extend through the split nut hole of the bottom plate, through the split nut, and continue through the corresponding split nut hole 234 in the top plate.

Figure 4A also illustrates the general direction of the rotational movement of the cam disc by arrow 992, and the general direction of the movement of the split nut away from and towards the main body by arrow 993, when the input force to the mechanism is applied and subsequently released, causing the cam to cause movement of the split nut. These movements are illustrated in further detail in the following figures. Arrow 991 illustrates the centripetal movement away from and the centripetal movement towards the center axis 242 of the cam disc.

FIGURE 4B illustrates the closed position of a gripping apparatus 100. The primary movement direction of the moveable handle 211 is indicated by arrow 995. In the closed position, the gripping apparatus is configured so that each jaw assembly is at a minimum distance from the center axis of the cam disc or from the centrally-located hole in the bottom plate. In the closed position, the jaw assemblies may be in physical contact with each other. While gripping a dispenser, the jaw assemblies may be in physical contact with each other or have spaces or gaps between them, depending on the size of the dispenser being gripped. While gripping a dispenser, at least one jaw assembly and at least one counterpart assembly may be in physical contact with each other or have spaces or gaps between them, depending on the size of the dispenser being gripped. The possible spaces or gaps in closed position are smaller than in the open position, as the jaw assemblies have moved towards the centrally-located hole of the bottom plate along the bottom plate grooves when transitioning from the open position towards the closed position.

In the closed position, the possible spaces or gaps between the jaw assemblies, or between the at least one jaw assembly and the at least one counterpart assembly, may be less than 3 cm, for example less than 2 cm, for example less than 1.5 cm, for example less than 1 cm, for example less than 0.8 cm, for example less than 0.6 cm, for example less than 0.4 cm, for example less than 0.2 cm, or for example less than 0.1 cm, or there may be no spaces or gaps.

In the open position, the spaces or gaps between the jaw assemblies, or between the at least one jaw assembly and the at least one counterpart assembly, may be for example from 0.1 cm to 100 cm, or from 0.1 cm to 10 cm, or from 0.1 to 5 cm, or from 0.1 to 3 cm, or from 0.1 to 2 cm, or from 0.1 to 1 cm, or from 0.1 to 0.5 cm.

Importantly, as illustrated by figures 4A and 4B, the apparatus self-centers (in the horizontal plane) the dispenser being gripped during the transition from the open position to the closed position. In other words, even if a dispenser is not set in the center of the gripper when open, the movement of jaws will ensure that the dispenser is centered when the closed position is reached. Further, turning to the vertical plane, the apparatus also levels the dispenser being gripped during the transition from the open position to the closed position. This may be achieved, for example, by using beveled or tilted surfaces 256 in the gripping apparatus. In other words, even if a dispenser is set at an angle when the apparatus is in the open position, the movement of the jaws will ensure that the dispenser is level, i.e., coplanar with the bottom plate, when the closed position is reached.

A gripping apparatus, such as gripping apparatus 100, may be configured to perform the self-centering and levelling concurrently, for example when the gripper state changes from the open position to the closed position. This is beneficial as the top surface of the dispenser, and consequently the dispenser, will then be aligned with the gripping apparatus in both the horizontal and vertical planes, increasing stability during movement and improving the predictability of the movements, for example the dispensing action.

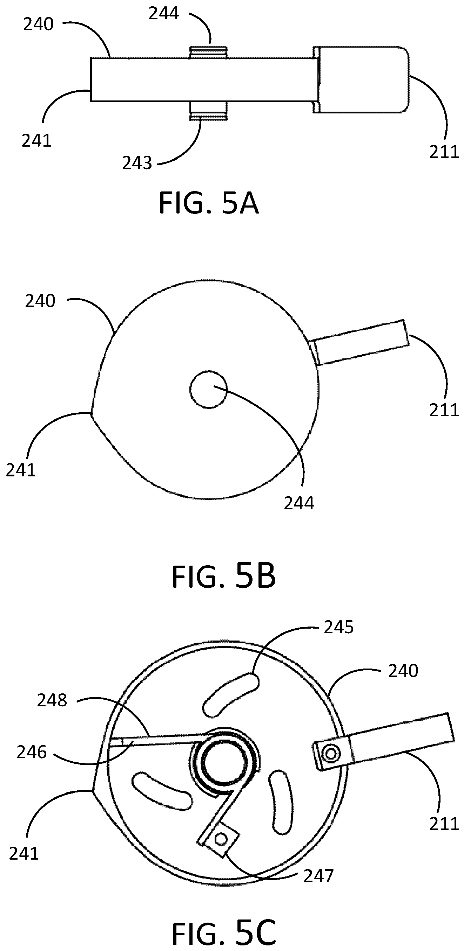

FIGURES 5A, 5B, and 5C illustrate an example of a cam disc suitable for use with any of the gripping apparatuses of the present disclosure. Figure 5A shows the cam disc from a side view. Figure 5B shows the cam disc from above. Figure 5C shows the cam disc from below.

In figure 5A, the cam disc 240, the moveable handle 211, the bottom protrusion 243, and the top protrusion 244 are illustrated from the side. The bottom and top protrusions are positioned in the middle of the cam disc, at the center axis.

In figure 5B, the cam disc 240 is shown from above. The cam disc is a disc that comprises at least one cam 241. The cam is an asymmetrical feature that protrudes horizontally from the periphery of the cam disc. The cam is positioned opposite to the moveable handle, so that an imaginary line from the moveable handle to the cam splits the cam disc in half. The cam is configured to move rotationally towards the split nut upon application of input force to the moveable handle.

In some embodiments, the moveable handle and the cam move the same distance rotationally upon application of the input force to the moveable handle. The cam may be fixedly attached to or an integral part of the cam disc. In some embodiments, there may be several cams in the cam disc. In some embodiments, there may be several moveable handles. The moveable handle and the at least one cam may be positioned at any suitable positions along the periphery of the cam disc.

In figure 5C, the cam disc is shown from below. The cam disc further comprises arched grooves 245, a spring housing 247, and a spring groove 248 on the underside of the cam disc. The arched grooves may be "banana-shaped". The arched grooves extend both in a centripetal direction and in a rotational direction, as viewed from the centre axis 242, and allow the plurality of jaw assemblies to move in a centripetal direction along the bottom plate grooves, while the cam disc simultaneously moves in a rotational direction. This is due to the arched grooves extending both horizontally and centripetally in a curved shape.

In some embodiments, the cam disc protrudes vertically downwards (bottom protrusion 243) through a centrally-located hole in the bottom plate, and optionally upwards (top protrusion 244) through a centrally-located hole in the top plate. In some embodiments, the cam disc does not protrude vertically.

The spring groove is a groove or a recessed feature on the underside of the cam disc, designed to accommodate a spring 246 within the spring housing 247. The spring 246 is wrapped at least partially around the central axis or it may be coiled in one or more full rotations around it. One end of the spring rests within the spring housing 247, while the other end is positioned at the other end of the spring groove 248, near the outer edge of the cam disc. The spring allows for smooth movement of the cam disc in a rotational direction.

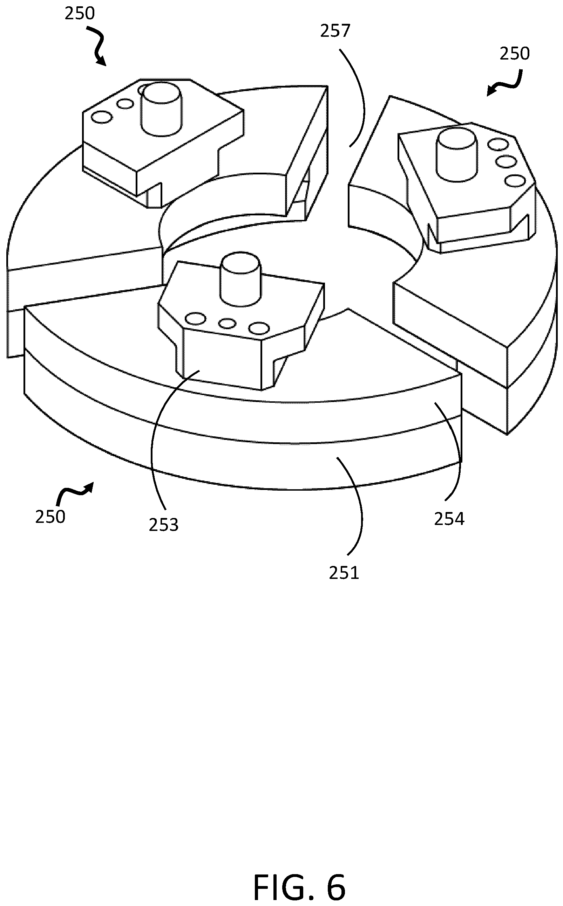

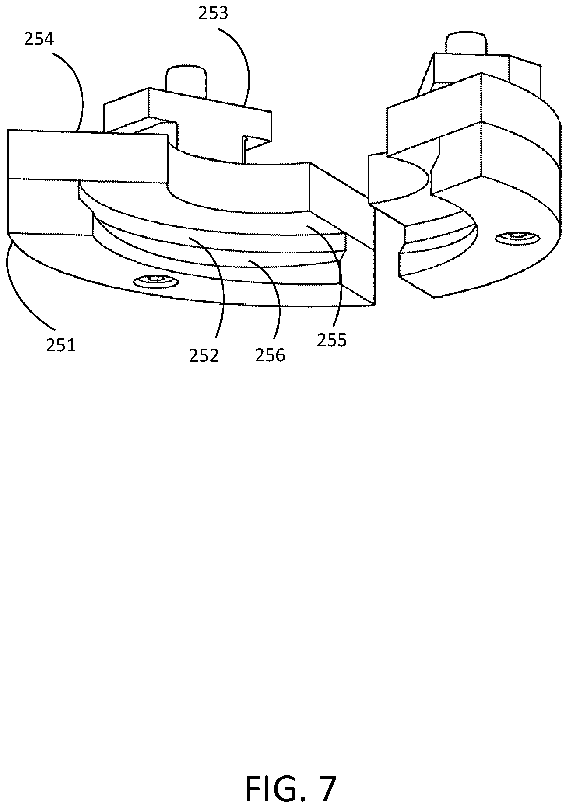

FIGURES 6 and 7 illustrate the jaw assemblies and parts thereof, namely the jaw, the spacer, and the jaw slider.

FIGURE 6 shows an exemplary embodiment of a plurality of jaw assemblies from an isometric top view which is capable of supporting at least some embodiments of the present disclosure. Figure 6 illustrates an embodiment with three jaw assemblies. As seen in figure 6, a jaw assembly 250 comprises a jaw 251, a jaw slider 253, and a spacer 254. A jaw gap 257 is also illustrated.

The apparatus comprises a plurality of jaw assemblies, for example two jaw assemblies. For example, three jaw assemblies may be used. This provides a benefit of providing gripping force from several directions, which improves stability and distribution of force on the workpiece being gripped. A larger number of jaw assemblies is also usable, for example 2, 3, 4, or 5 and up to 12 jaw assemblies.

The outer edges of a jaw and a spacer may be curved. Similarly, the inner edges may be curved. As a result, the plurality of jaw assemblies may collectively form a generally circular shape with their outer edges and another, smaller circular shape with their inner edges. The inner circle is concentric with and smaller than the outer circle. Alternatively, the jaws may have other geometries, such as rectangular, or irregular shapes, depending on the specific gripping requirements. For a circular dispenser being gripped, such as a plunger flange of a syringe, a generally circular jaw assembly may be suitable.

The jaw 251 is a part of the jaw assembly configured to engage with and directly contact a dispenser, for example a syringe plunger flange. Each jaw comprises a tilted surface 256 on the inner side of the jaw, the inner side being the side that is closer to the centre axis.

The spacer 254 is attached to the jaw 251. The spacer connects the jaw to the jaw slider and is also configured to provide sufficient vertical space for the dispenser, for example the syringe plunger flange, to fit between the tilted surface of the jaw and the bottom surface of the spacer.

The jaw slider 253 protrudes upwards from the spacer, through the bottom plate groove 222. The jaw slider is configured to fit the bottom plate groove of a gripping apparatus, for example gripping apparatus 100. The jaw slider is configured to move centripetally away from the centre axis along the bottom plate groove upon application of input force to the moveable handle, thereby moving the whole jaw assembly, for example, at least the jaw, the spacer, and the jaw slider with respect to the gripping apparatus.

FIGURE 7 shows two jaw assemblies from an isometric bottom view. The tilted surface 256 of the jaw 251 and the bottom surface 255 of the spacer are shown. The space or cavity between the tilted surface of the jaw and the bottom surface 255 of the spacer, is designed to fit and/or interlock the dispenser, for example the syringe plunger flange when the gripped by the jaws. Above the tilted surface is the inner side 252 of the jaw. When placing a dispenser in the gripping apparatus, it is the inner side 252 that contacts the dispenser from the side, when the dispenser is placed against the bottom surface 255 of the spacer, which contacts the top surface of the dispenser. Further, a gap between the jaw assemblies is illustrated. In general, a gap is formed between two jaw assemblies when the jaws move towards the open position. In the closed position, the jaw assemblies may be in contact with each other and there may not be a gap. When a dispenser, for example a syringe 400, is interlocked in the cavity between the jaws, there may be small gaps between the jaw assemblies, or there may be no gaps, depending on the size of the syringe.

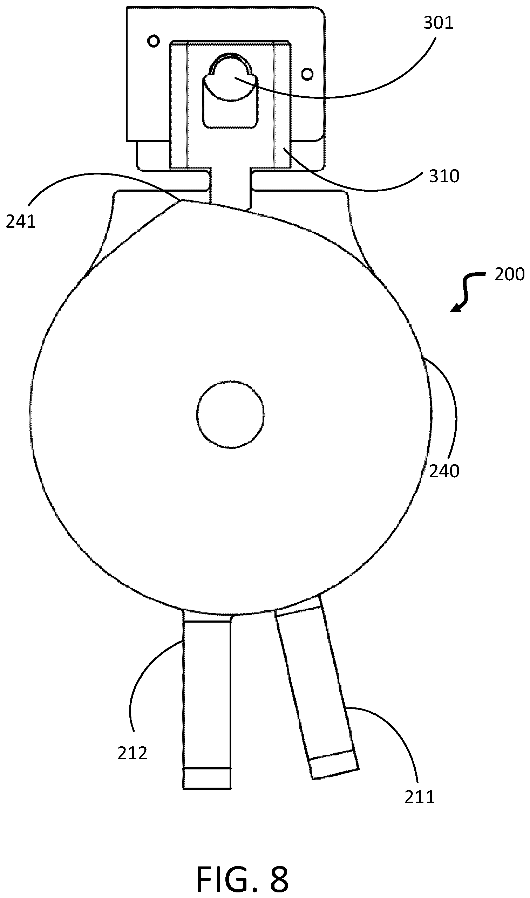

FIGURE 8 illustrates an exemplary embodiment of a cam disc 240 and a split nut 310. Cam 241 is shown in relation to the split nut 310. As an input force is applied to the moveable handle 211, the cam disc rotates, thereby causing the cam to move towards the split nut and physically contact the split nut, thereby causing the split nut to move away from the main body 200. As the split nut moves, the split nut shifts away from the leadscrew which extends through the split nut central hole 301, thereby disengaging the leadscrew. This disengagement allows for the rapid vertical movement (rapid traverse) of the gripping apparatus. A user or automated device may easily move the apparatus vertically while applying pressure to the moveable handle, without the movement being inhibited by the leadscrew or any actuator connected to it. Should the user wish to move the dispenser along with the apparatus while quick traversing, the user may use their other hand to hold the dispenser in place during the movement. Upon releasing the pressure on the handle, for example at the end of the traverse movement, the dispenser will be (again) securely attached to the apparatus.

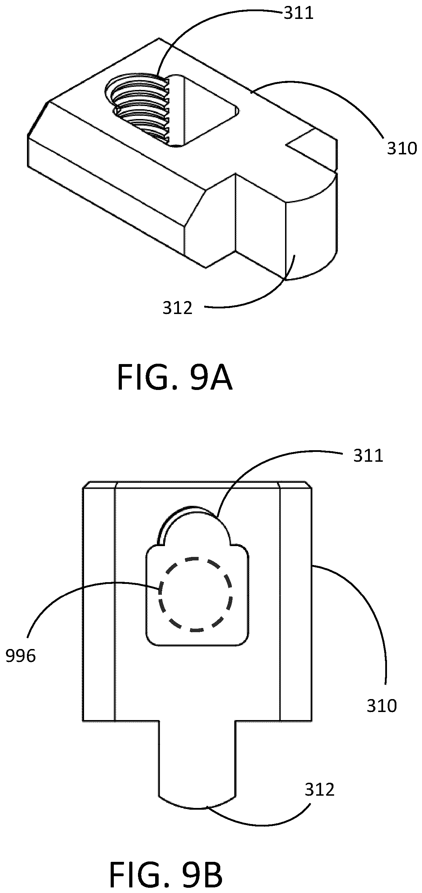

FIGURE 9A shows an exemplary embodiment of a split nut from an isometric view. The split nut 310 is a half of a screw nut, i.e., a screw nut split in half. The split nut further comprises a slide protrusion 312. The split nut functions as a slider via the slide protrusion, by sliding away from the leadscrew upon application of input force to the moveable handle which causes the cam to actuate the split nut by rotating towards the slide protrusion. Release of said input force causes the split nut to slide towards the leadscrew, thereby engaging the split nut to the leadscrew. Unlike a conventional screw nut, the split nut features screw grooves, for example a female thread portion, on only one side, forming a half-circle 311. When the gripping apparatus is in the closed position, the screw grooves of the split nut engage with the leadscrew, thereby securing the gripping apparatus to the leadscrew. Other alternatives for the cam function and the split nut slide protrusion include spring and/or lever constructions which are configured push the split nut away. When the gripping apparatus is in the open position, the split nut comprising a half-circle with grooves is positioned away from the leadscrew. Therefore, when the apparatus is in the open position, the apparatus can be moved along the leadscrew in a vertical direction without the leadscrew turning or engaging with the grooves. When the apparatus is in the closed position, the split nut comprising the half-circle with screw grooves prevents vertical movement along the leadscrew. The split nut allows for quick attachment and detachment of the apparatus to the leadscrew.

FIGURE 9B illustrates a split nut from a top view. Leadscrew axis 996 is illustrated in the opening of the split nut 310. The axis 996 illustrates where the leadscrew is positioned when the apparatus 100 is in the open position. When the apparatus 100 moves towards the closed position, the split nut moves back towards the axis, and the half circle with screw grooves 311 engage with the leadscrew.

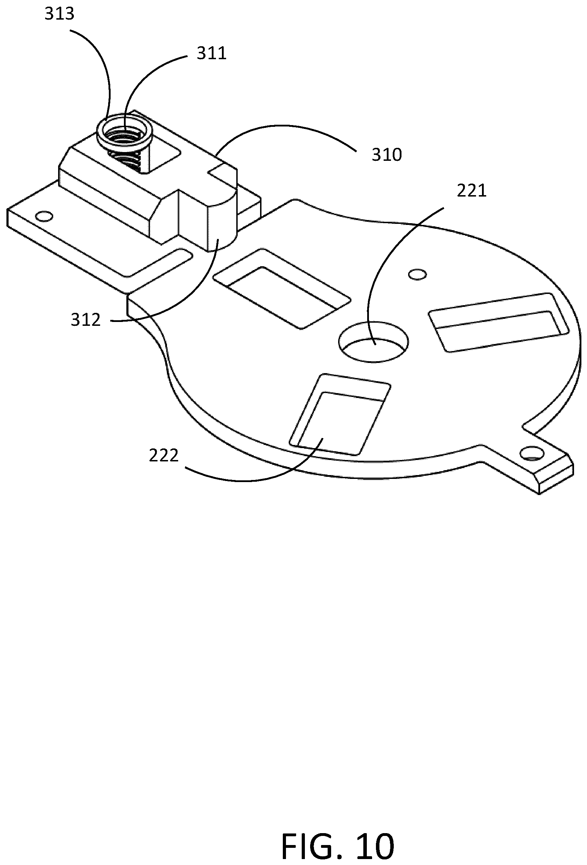

FIGURE 10 illustrates an exemplary embodiment of a split nut 310 on a bottom plate 220 from an isometric top view. The half-circle of screw grooves 311 located on the split nut is illustrated. Additionally, a retaining ring 313 is illustrated, positioned on top of the split nut. The retaining ring 313 keeps the leadscrew axis fixed in its position, while allowing the split nut to move in a horizontal direction. The retaining ring may be attached to the top plate, the bottom plate or other components of a gripping apparatus. Generally, a first and second retaining ring are used, where the first retaining ring is above the split nut and the second one is below the split nut.

The split nut moves away from the main body upon application of an input force to the mechanism, for example to the moveable handle of the handle assembly, which causes the cam to rotate towards one end of the split nut, and disengages the part of the split nut that comprises the half-circle with screw grooves from the leadscrew. The split nut engages to the leadscrew when an input force to the mechanism, for example to the moveable handle of the handle assembly, is released, for example, when the apparatus is in closed position.

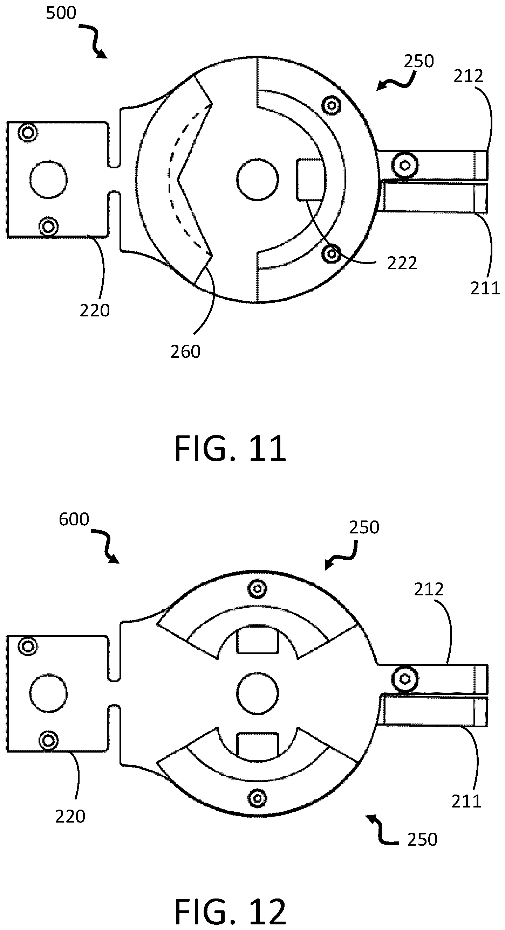

FIGURE 11 illustrates a bottom view of an example apparatus 500 capable of supporting at least some embodiments of the present disclosure. In figure 11, a single-jaw solution is illustrated. In this embodiment, the gripping apparatus comprises one jaw assembly 250, and one stationary counterpart assembly 260. When gripping a dispenser, the dispenser can become fixed in an interlocked position between the at least one jaw of the jaw assembly and the stationary counterpart assembly 260. Apparatus 500 is usable with cam 240 and split nut 310 as shown for example in figures 5, 8, and 9.

FIGURE 12 illustrates a bottom view of an example apparatus 600 capable of supporting at least some embodiments of the present disclosure. In figure 12, a double-jaw solution is illustrated. In this embodiment, the gripping apparatus comprises two jaw assemblies 250. When gripping a dispenser, the dispenser can become fixed in an interlocked position between the two jaws of the two jaw assemblies. Apparatus 600 is usable with cam 240 and split nut 310 as shown for example in figures 5, 8, and 9.

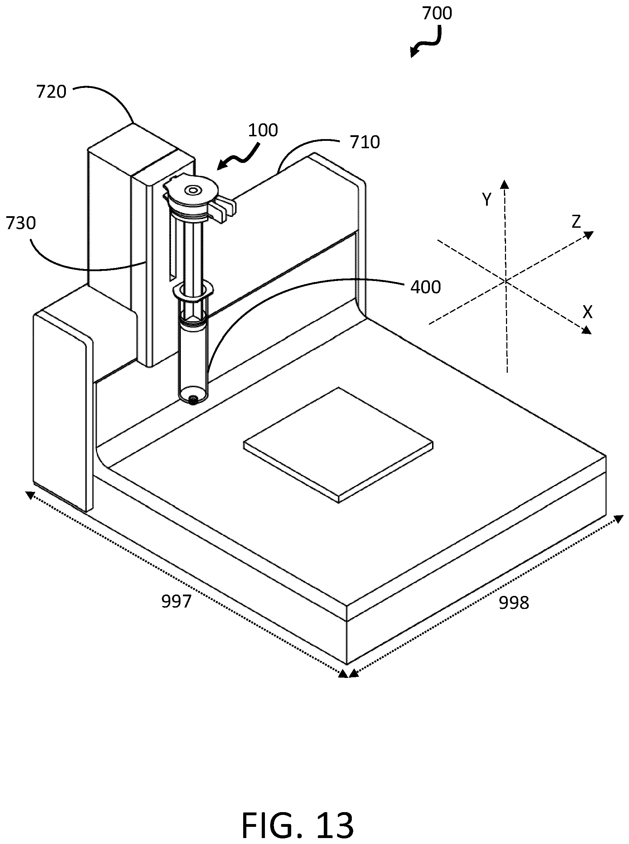

FIGURE 13 illustrates an isometric top view of a liquid handling apparatus capable of supporting at least some embodiments of the present disclosure. The liquid handling apparatus 700 comprises a bridge assembly 710 and a drive mechanism 720. The liquid handling apparatus 700 further comprises a leadscrew, an actuator, a linear guide block, and a linear guide rail. At least some of the components of apparatus 700 may be located within the housing 730 of the apparatus.

The liquid handling apparatus 700 comprises an end-effector, for example a gripping apparatus, in particular any one of gripping apparatuses 100, 500, 600. In figure 13, the apparatus is shown with exemplary gripping apparatus 100.

The liquid handling apparatus 700 is configured so that the end-effector is movable in XYZ directions, i.e. the horizontal, vertical and depth directions. The liquid handing apparatus 700 is also programmable to operate autonomously. Consequently, the liquid handling apparatus is a cartesian coordinate robot.

Vertical movement of the end-effector is provided by the leadscrew, the actuator and by a linear guide block. The leadscrew is an elongated threaded shaft configured to convert rotational motion into linear motion. The leadscrew is configured to engage with the corresponding threaded split nut for the purpose of driving and/or positioning the end-effector of the liquid handling apparatus in a vertical direction. The actuator is a device configured to provide controlled rotational motion for driving a mechanical element or system with precise positioning, for example a stepper motor. The actuator is configured to actuate the end-effector of the liquid handling apparatus along a vertical axis via the leadscrew. The linear guide block is configured to move linearly along a guide rail, wherein the linear guide block comprises a housing having rolling elements, such as balls or rollers, arranged to facilitate linear motion with minimal friction between the guide block and the guide rail. The linear guide block serves as the part connecting the apparatus to the linear guide rail. The linear guide rail comprises an elongated, rigid structural member having at least one guide surface or raceway, configured to guide and support the linear guide block during linear motion. The linear guide block/rail combination may be used together with the leadscrew to provide smooth movement of the end-effector, including prevention of excess rotation, Further, when the leadscrew is selectably detachable, rapid traverse functionality may be provided as discussed above.

Horizontal movement (Z-axis) of the end-effector is provided by moving drive mechanism 720 along the bridge assembly 710. Liquid handling apparatus 700 may be configured to use a further leadscrew, further actuator and a further linear guide block/rail combination to move the entire drive mechanism 720 in either direction along bridge 710. The further leadscrew, further actuator, and further linear guide block/rail combination may be located within bridge assembly 710 of the apparatus. Thereby, drive mechanism 720 may be moved in either direction along working distance 998.

A third axis, i.e., the depth axis, of movement (X-axis) is provided by moving bridge assembly 710, and thereby drive mechanism 720, along the length of the working area 997. Liquid handling apparatus 700 may be configured to use a further leadscrew, further actuator and a further a linear guide block/rail combination to move bridge assembly 710 in either direction along working distance 997.

In some embodiments, the main body is configured to disengage the split nut from a leadscrew when a force is applied to the handle assembly. In some embodiments, the apparatus is configured to simultaneously grip the dispenser and disengage the split nut from the leadscrew when the force is applied to the handle assembly. The split nut is disengaged with the help of arched grooves configured to facilitate rotational movement of the cam disc, located on the underside of the cam disc, whereby the rotational movement causes a split nut to move away from a leadscrew, thereby disengaging the split nut from the leadscrew.

In an embodiment, the handle assembly may comprise more than one moveable handle. In an embodiment, neither handle in the handle assembly is stationary. In an embodiment, the handle assembly may be configured to receive a vertically-directed input force. In an embodiment, the handle assembly may comprise a button or a trigger, wherein the button or trigger may be configured to convey force to the cam disc. The force conveyed to the cam disc may be equal, smaller, or greater than the force required to press the button or trigger.

In some embodiments, the dispenser may be rotated by a user or an automated device whilst being interlocked vertically and horizontally in the cavity in the closed position.

In some embodiments, the apparatus is configured to grip a plunger flange of a syringe, and the main body is configured to move the plurality of jaw assemblies away from the center of the cam disc upon application of force to the handle assembly.

In some embodiments, the apparatus is configured to disengage the split nut from a leadscrew when a force is applied to the handle assembly. In some embodiments, the apparatus is configured to simultaneously release the dispenser and disengage the split nut from the leadscrew when the force is applied to the handle assembly. The apparatus may be configured to simultaneously grip a dispenser and engage a split nut to a leadscrew when an input force is released.

In some embodiments, the apparatus for gripping a dispenser forms a part of a liquid handling apparatus. The liquid handling apparatus comprises at least the apparatus for gripping a dispenser and an actuator configured to actuate the apparatus for gripping a dispenser via a leadscrew along a vertical axis, for example, in the z-direction within an x-y-z coordinate system.

In some embodiments, the liquid handling apparatus further comprises a controller operatively connected to the liquid handling apparatus, wherein the controller comprises at least one processing core, at least one memory including computer program code, the at least one memory and the computer program code being configured to, with the at least one processing core, cause the controller to control movement of the liquid handling apparatus. The liquid handling apparatus may further comprise a screen, wherein the screen comprises a user interface configured to at least participate in controlling the movements of the liquid handling apparatus.

One type of controller may comprise, for example, at least one computing device such a server, node or cloud computing device. Comprised in the computing device is a processor, which may comprise, for example, a single- or multi-core processor wherein a single-core processor comprises one processing core and a multi-core processor comprises more than one processing core. The processor may comprise more than one processor. A processing core may comprise, for example, a Cortex-A8 processing core by ARM Holdings or a Steamroller processing core produced by Advanced Micro Devices Corporation. The processor may comprise at least one Qualcomm Snapdragon and/or Intel Core processor, for example. The processor may comprise at least one application-specific integrated circuit, ASIC. The processor may comprise at least one field-programmable gate array, FPGA. The processor may be a means for performing method steps in the computing device. The processor may be configured, at least in part by computer instructions, to perform actions, for example to control movements of the liquid handling apparatus by controlling actuators connected to the apparatus.

In the context of the present disclosure, it is understood that the controller may comprise several devices configured to act in cooperation. The devices may interface via a network used to facilitate communication to and from controllers, measurement devices, meters, motors, gantries and other elements. Usable network technologies comprise: wireless local area network, WLAN, Ethernet, universal serial bus, USB, and/or worldwide interoperability for microwave access, WiMAX, standards, and satellite communication methods, direct wiring such as electrical wires, for example. In some embodiments, CANbus may be used, wherein CANbus may include an ISO 11898-2 high speed CANbus.

In some embodiments, a programmable logic controller, PLC, may be used as the controller. The standard IEC 61131 defines programmable logic controllers, however, in the present disclosure the term "PLC" is not limited to the standard. The PLC may be connected to the devices by a variety of means, including but not limited to electrical wires.

The controller may comprise or be connected to at least one of the following: a personal computer, a server, a mobile phone, a smartphone, a tablet device, a smart watch, an item of smart jewellery or any type of suitable electronic device.

This disclosure further presents a method for gripping a dispenser. In some embodiments, the method comprises:
applying a force to a movable handle of an apparatus, such as gripping apparatus 100, the apparatus configured so that at least two jaw assemblies move centripetally away from the center of a cam disc upon application of said force; wherein each jaw assembly comprises a jaw, a spacer, and a jaw slider;
placing the dispenser against the underside of each spacer within an area defined by the at least two jaw assemblies; and
releasing the force on the movable handle, the apparatus configured so that the releasing of the force moves the at least two jaw assemblies centripetally towards the center of the cam disc, thereby fixing and self-centering the dispenser in a cavity within the at least two jaw assemblies, whereby horizontal and vertical movement of the syringe is prevented.

In some embodiments, the applying of a force comprises applying a horizontally-directed input force from the side of the moveable handle, wherein said horizontally-directed input force is applied by a user or by an automated device. In some embodiments, the moveable handle may be configured to receive a vertically-directed input force. An input force may be conveyed in any suitable direction that causes the moveable handle to actuate the main body of the gripping apparatus.

The method for gripping a syringe may further comprise removing the syringe from the apparatus by applying an input force to the mechanism, such as a horizontal force to the moveable handle, after the releasing of the force.

The method for gripping a dispenser may further comprise moving the apparatus along a vertical axis via a leadscrew.

A device operatively connected to the liquid handling apparatus, wherein the device comprises at least one processing core, at least one memory including computer program code, the at least one memory and the computer program code being configured to, with the at least one processing core, cause the device to control movement of the liquid handling apparatus.

Advantages of the present disclosure include improved dispenser attachment and detachment, self-centering of the dispenser in two dimensions, and high robustness of the apparatus. Additional advantages include reduced play and reduced clearance drift, because of the dispenser being interlocked in a fixed position when the apparatus is in closed position. This advantage also leads to the advantage of avoiding air from entering the dispenser, thereby avoiding air bubbles in the material to be dispensed. This is because if the dispenser was not interlocked in a fixed position, movement of the dispenser could occur, for example movement of a plunger flange of a syringe, which could potentially cause the dispenser to aspirate air into the dispenser, which could be detrimental, for example by reducing the measurement accuracy of the material to be dispensed.

The embodiments disclosed provide a technical solution to a technical problem. One technical problem being solved is eliminating the need for two separate mechanisms to move an apparatus vertically and to release a dispenser being gripped. The apparatus of the present disclosure allows for simultaneous release of a dispenser and disengagement of the split nut, which allows vertical movement, with an input force to a single handle. Typically, a separate mechanism is needed to allow vertical movement. The apparatus of the present disclosure allows for simultaneous gripping of a dispenser and engagement of a split nut to a leadscrew, by applying and subsequently releasing an input force to a single handle.

The present disclosure can also be utilized via the following clauses:
Clause 1. A method of attaching a dispenser, for example a syringe and/or a cartridge, to a liquid handling apparatus, the method comprising: applying a force to a movable handle of an gripper apparatus, the gripper apparatus configured so that at least one jaw assembly moves centripetally away from the center of a cam disc upon application of said force; wherein each jaw assembly comprises a jaw, a spacer, and a slider; placing a top surface of the dispenser within the gripper apparatus against the underside of each spacer within an area defined by the at least one jaw assembly; and releasing the force on the movable handle, the apparatus configured so that the releasing of the force moves the at least one jaw assembly centripetally towards the center of the cam disc, thereby fixing the dispenser to the gripper apparatus in a cavity within the at least one jaw assembly, whereby horizontal and vertical movement of the dispenser is prevented.
Clause 2. The method of clause 1, wherein the applying of a force simultaneously disengages a split nut from a leadscrew, thereby allowing movement, for example rapid traverse, of the gripper apparatus along the leadscrew.
Clause 3. A liquid handling apparatus comprising: a gripping apparatus comprising: a main body comprising a cam disc, a bottom plate, and at least one jaw assembly, a split nut, a split nut body adjacent to the main body, said split nut body configured to house the split nut, and a handle assembly fixedly attached to the main body, each jaw assembly comprising a jaw slider, a spacer, and a jaw; and an actuator configured to actuate the gripping apparatus along a vertical axis via a leadscrew, wherein the gripping apparatus is configured to receive at least a part of a dispenser, for example a syringe and/or a cartridge within the gripping apparatus and fix said part detachably to the gripping apparatus, wherein the liquid handling apparatus is configured to move along a horizontal axis in both x- and y-directions within an x-y coordinate system, the liquid handling apparatus further comprising a controller operatively connected to the liquid handling apparatus, wherein the controller comprises at least one processing core, at least one memory including computer program code, the at least one memory and the computer program code being configured to, with the at least one processing core, cause the controller to control movement of the liquid handling apparatus along at least one of the horizontal and/or vertical axis.
Clause 4. A non-transitory computer readable medium having stored thereon a set of computer readable instructions that, when executed by at least one processor, cause an controller to at least to control movement of a liquid handling apparatus in accordance with clause 3 along at least one of the horizontal and/or vertical axis.
Clause 5. An apparatus for gripping a syringe, the apparatus comprising: a main body comprising a cam disc, a bottom plate, and at least one jaw assembly, a split nut, a split nut body adjacent to the main body, said split nut body configured to house the split nut, and a handle assembly fixedly attached to the main body, each jaw assembly comprising a jaw slider, a spacer, and a jaw.
Clause 6. The apparatus of clause 5, wherein the apparatus is configured to grip a plunger flange of the syringe, wherein the apparatus is configured so that the at least one jaw assembly is moved away from the center of the cam disc upon application of force to the cam disc, for example where the force is applied to the cam disc via the handle assembly.
Clause 7. The apparatus of clause 5 or clause 6, wherein apparatus is configured so that the split nut is disengaged from a leadscrew when a force is applied to the handle assembly, wherein the leadscrew is configured to at least participate in actuating the apparatus in a vertical direction when the split nut is engaged to said leadscrew.
Clause 8. The apparatus of any one of clauses 5 to 7, wherein the apparatus is configured so that the syringe flange is released from the apparatus, and the split nut is disengaged from the leadscrew, when the force is applied to the handle assembly.
Clause 9. The apparatus of any one of clauses 5 to 8, wherein the apparatus comprises at least three jaw assemblies, wherein the apparatus is configured to grip the syringe flange within a space between said three jaw assemblies.
Clause 10. The apparatus of any one of clauses 5 to 9, wherein the handle assembly comprises a moveable handle and a stationary handle, wherein the moveable handle is fixedly attached to the cam disc, and wherein the stationary handle is fixedly attached to the apparatus, for example via the bottom plate or to the top plate.
Clause 11. An apparatus for gripping a dispenser, the apparatus comprising: a main body comprising a cam disc, a bottom plate, and at least one jaw assembly, a split nut, a split nut body adjacent to the main body, said split nut body configured to house the split nut, and a handle assembly fixedly attached to the main body, each jaw assembly comprising a jaw slider, a spacer, and a jaw.
Clause 12. The apparatus of clause 11, wherein the apparatus is configured to grip a dispenser, for example a movable part of the dispenser, in particular a plunger flange of a syringe, wherein the main body is configured to move the at least one jaw assembly away from the center of the cam disc upon application of force to the cam disc, for example where the force is applied to the cam disc via the handle assembly.
Clause 13. The apparatus of clause 11 or clause 12, wherein the gripping comprises fixing the dispenser between the spacer and a tilted surface of the jaw.
Clause 14. The apparatus of any one of clauses 11 to 13, wherein the main body comprises at least two jaw assemblies; wherein the apparatus has a closed position, wherein the at least two jaw assemblies are arranged to contact each other; and an open position, wherein the at least two jaw assemblies define a space between them.
Clause 15. The apparatus of any one of clauses 11 to 14, wherein the main body is configured to disengage the split nut from a leadscrew when a force is applied to the handle assembly.
Clause 16. The apparatus of any one of clauses 11 to 15, wherein the apparatus is configured to simultaneously release the dispenser and disengage the split nut from the leadscrew when the force is applied to the handle assembly.
Clause 17. The apparatus of any one of clauses 11 to 16, wherein the main body comprises at least three jaw assemblies.
Clause 18. The apparatus of any one of clauses 11 to 17, wherein the main body further comprises a top plate.
Clause 19. The apparatus of any one of clauses 11 to 18, wherein the handle assembly comprises a moveable handle and a stationary handle, wherein the moveable handle is fixedly attached to the cam disc, and wherein the stationary handle is fixedly attached to the bottom plate or to the top plate.
Clause 20. The apparatus of any one of clauses 11 to 19, wherein the force comprises an input force conveyed in a suitable direction, such as a horizontally-directed input force, to the moveable handle of the handle assembly.
Clause 21. The apparatus of any one of clauses 11 to 20, wherein the apparatus is configured to be connected to a linear actuator via the leadscrew.
Clause 22. The apparatus of any one of clauses 11 to 21, wherein the underside of the cam disc comprises arched grooves configured to facilitate rotational movement of the cam disc, wherein the rotational movement of the cam disc causes the cam to come into contact with and actuate the split nut away from the leadscrew, thereby disengaging the split nut from the leadscrew.
Clause 23. The apparatus of any one of clauses 11 to 22, wherein the bottom plate comprises at least one groove configured to allow centripetal movement of at least one respective jaw slider.
Clause 24. The apparatus of any one of clauses 11 to 23, further comprising a housing configured to protect the apparatus.
Clause 25. The apparatus of any one of clauses 11 to 24, wherein the at least one jaw is configured to move centripetally away from the center of the cam disc, thereby releasing the dispenser, upon application of force to the handle assembly.
Clause 26. The apparatus of any one of clauses 11 to 25, wherein the at least one jaw comprises a tilted surface, wherein the tilted surface is configured to interlock with the dispenser.
Clause 27. The apparatus of clause 26, wherein the dispenser is interlocked between the upper edge of the tilted surface of the jaw and the bottom surface of the spacer.
Clause 28. The apparatus of any one of clauses 11 to 27, wherein the cam disc comprises at least one protrusion, wherein the at least one protrusion extends through an opening of the bottom plate.
Clause 29. The apparatus of any one of clauses 11 to 28, further comprising a spring arranged around the center axle of the cam disc, wherein one end of the spring is fixedly attached within a spring housing of the cam disc, and another end of the spring is positioned adjacent to the outer edge of the cam disc.
Clause 30. A liquid handling apparatus comprising at least the apparatus of clause 11, and an actuator configured to actuate the apparatus of clause 11 along a vertical axis via a leadscrew.
Clause 31. The liquid handling apparatus of clause 30, wherein the liquid handling apparatus is configured to move along a horizontal axis in both x- and y-directions within an x-y coordinate system.
Clause 32. The liquid handling apparatus of any one of clauses 30 to 31, further comprising a device operatively connected to the liquid handling apparatus, wherein the device comprises at least one processing core, at least one memory including computer program code, the at least one memory and the computer program code being configured to, with the at least one processing core, cause the device to control movement of the liquid handling apparatus.
Clause 33. The liquid handling apparatus of any one of clauses 30-32, the liquid handling apparatus further comprising a screen, wherein the screen comprises a user interface configured to at least participate in controlling the movement of the liquid handling apparatus.
Clause 34. A method of gripping a dispenser, the method comprising: applying a force to a movable handle of an apparatus, the apparatus configured so that at least one jaw assembly moves centripetally away from the center of a cam disc upon application of said force; wherein each jaw assembly comprises a jaw, a spacer, and a jaw slider; placing a top surface of the dispenser against the underside of each spacer within an area defined by the at least one jaw assembly; and releasing the force on the movable handle, the apparatus configured so that the releasing of the force moves the at least one jaw assembly centripetally towards the center of the cam disc, thereby fixing the dispenser in a cavity within the at least one jaw assembly, whereby horizontal and vertical movement of the dispenser is prevented.
Clause 35. The method according to clause 34, the method further comprising removing the dispenser from the apparatus by applying a force to the moveable handle after the releasing of the force.
Clause 36. The method according to clause 35, said applying comprising applying an input force to the moveable handle, wherein said input force is applied by a user or by an automated device.
Clause 37. The method of any one of clauses 34 to 36, the method further comprising moving the apparatus along a vertical axis via a leadscrew.
Clause 38. The method of any one of clauses 34 to 37, wherein the applying of a force simultaneously disengages a split nut from a leadscrew, thereby allowing movement along a vertical axis via the leadscrew.
Clause 39. The method of any one of clauses 34 to 38, wherein the releasing of a force simultaneously engages the split nut to the leadscrew, thereby securing the apparatus to the leadscrew and preventing movement along a vertical axis.

It is understood that variations and combinations in accordance with the present disclosure which relate to groove dimensioning are within the understanding of the skilled artisan.

It is to be understood that the embodiments of the invention disclosed are not limited to the particular structures, process steps, or materials disclosed herein, but are extended to equivalents thereof as would be recognized by those ordinarily skilled in the relevant arts. It should also be understood that terminology employed herein is used for the purpose of describing particular embodiments only and is not intended to be limiting.

Reference throughout this specification to one embodiment or an embodiment means that a particular feature, structure, or characteristic described in connection with the embodiment is included in at least one embodiment of the present invention. Thus, appearances of the phrases "in one embodiment" or "in an embodiment" in various places throughout this specification are not necessarily all referring to the same embodiment. Where reference is made to a numerical value using a term such as, for example, about or substantially, the exact numerical value is also disclosed.

As used herein, a plurality of items, structural elements, compositional elements, and/or materials may be presented in a common list for convenience. However, these lists should be construed as though each member of the list is individually identified as a separate and unique member. Thus, no individual member of such list should be construed as a de facto equivalent of any other member of the same list solely based on their presentation in a common group without indications to the contrary. In addition, various embodiments and examples of the present invention may be referred to herein along with alternatives for the various components thereof. It is understood that such embodiments, examples, and alternatives are not to be construed as de facto equivalents of one another, but are to be considered as separate and autonomous representations of the present invention.

Furthermore, the described features, structures, or characteristics may be combined in any suitable manner in one or more embodiments. In this description, numerous specific details are provided, such as examples of lengths, widths, shapes, etc., to provide a thorough understanding of embodiments of the invention. One skilled in the relevant art will recognize, however, that the invention can be practiced without one or more of the specific details, or with other methods, components, materials, etc. In other instances, well-known structures, materials, or operations are not shown or described in detail to avoid obscuring aspects of the invention.

For the purposes of the present disclosure, the phrases "A or B" and "A and/or B" means (A), (B), or (A and B). For the purposes of the present disclosure, the phrase "A, B, and/or C" means (A), (B), (C), (A and B), (A and C), (B and C), or (A, B, and C).

While the forgoing examples are illustrative of the principles of the present invention in one or more particular applications, it will be apparent to those of ordinary skill in the art that numerous modifications in form, usage and details of implementation can be made without the exercise of inventive faculty, and without departing from the principles and concepts of the invention. Accordingly, it is not intended that the invention be limited, except as by the claims set forth below.

The verbs "to comprise" and "to include" are used in this document as open limitations that neither exclude nor require the existence of also un-recited features. The features recited in depending claims are mutually freely combinable unless otherwise explicitly stated. Furthermore, it is to be understood that the use of "a" or "an", that is, a singular form, throughout this document does not exclude a plurality.

### INDUSTRIAL APPLICABILITY

At least some embodiments of the present invention find industrial application in liquid handling apparatuses, more specifically in dispenser attachment, detachment, and controlled movement within such apparatuses.

**REFERENCE SIGNS LIST**

| | |
|---|---|
| 100 | Gripping apparatus |
| 200 | Main body |
| 210 | Handle assembly |
| 211 | Moveable handle |
| 212 | Stationary handle |
| 220 | Bottom plate |
| 221 | Bottom plate hole |
| 222 | Bottom plate groove |
| 223 | Bottom plate bridge |
| 224 | Bottom plate gap |
| 225 | Bottom plate split nut hole |
| 230 | Top plate |
| 231 | Top plate hole |
| 232 | Top plate bridge |
| 233 | Top plate gap |
| 234 | Top plate split nut hole |
| 240 | Cam disc |
| 241 | Cam |
| 242 | Center axis |
| 243 | Bottom protrusion |
| 244 | Top protrusion |
| 245 | Arched groove |
| 246 | Spring |
| 247 | Spring housing |
| 248 | Spring groove |
| 250 | Jaw assembly |
| 251 | Jaw |
| 252 | Inner side of jaw |
| 253 | Jaw slider |
| 254 | Spacer |
| 255 | Bottom surface of spacer |
| 256 | Tilted surface of jaw |
| 257 | Jaw gap |
| 260 | Stationary assembly |
| 300 | Split nut body |
| 301 | Split nut central hole |
| 310 | Split nut |
| 311 | Half-circle with screw grooves |
| 312 | Slide protrusion |
| 313 | Retaining ring |
| 350 | Linear guide block |
| 351 | Linear guide block groove |
| 400 | Syringe |
| 401 | Barrel |
| 402 | Barrel flange |
| 403 | Plunger |
| 404 | Plunger flange |
| 405 | Seal |
| 406 | Luer lock |
| 407 | Luer slip |
| 500 | Gripping apparatus |
| 600 | Gripping apparatus |
| 700 | Liquid handling apparatus |
| 710 | Bridge assembly |
| 720 | Drive mechanism |
| 730 | Housing |
| 991 | Arrow indicating centripetal direction |
| 992 | Arrow indicating rotational direction |
| 993 | Arrow indicating split nut movement |
| 994 | Volume |
| 995 | Arrow indicating moveable handle movement |
| 996 | Leadscrew axis |
| 997 | Working distance |
| 998 | Working distance |

## Claims

1. An apparatus (100, 500, 600) for gripping a dispenser, the apparatus (100, 500, 600) comprising:
- a main body (200) comprising a cam disc (240), a bottom plate (220), and at least one jaw assembly (250),
- a split nut (310),
- a split nut body (300) adjacent to the main body (200), said split nut body (300) configured to house the split nut (310), and
- a handle assembly (210) fixedly attached to the main body (200),
each jaw assembly (250) comprising a jaw slider (253), a spacer (254), and a jaw (251).

2. The apparatus (100, 500, 600) of claim 1, wherein the apparatus (100, 500, 600) is configured to grip a dispenser, for example a movable part of the dispenser, in particular a plunger flange (404) of a syringe (400), wherein the main body (200) is configured to move the at least one jaw assembly (250) away from the center of the cam disc (240) upon application of force to the cam disc (240), for example where the force is applied to the cam disc (240) via the handle assembly (210).

3. The apparatus (100, 500, 600) of any one of the preceding claims, wherein the gripping comprises fixing the dispenser between the spacer (254) and a tilted surface (256) of the jaw (251).

4. The apparatus (100, 600) of any one of the preceding claims, wherein the main body (200) comprises at least two jaw assemblies (250), preferably at least three jaw assemblies (250); wherein the apparatus (100, 600) has a closed position, wherein the at least two jaw assemblies (250), preferably at least three jaw assemblies (250), are arranged to contact each other; and an open position, wherein the at least two jaw assemblies (250), preferably at least three jaw assemblies (250), define a space between them.

5. The apparatus (100, 500, 600) of any one of the preceding claims, wherein the main body (200) is configured to disengage the split nut (310) from a leadscrew when a force is applied to the handle assembly (210), wherein the force comprises an input force conveyed in a suitable direction, such as a horizontally-directed input force, to a moveable handle (211) of the handle assembly (210).

6. The apparatus (100, 500, 600) of any one of the preceding claims, wherein the apparatus (100, 500, 600) is configured to simultaneously release the dispenser and disengage the split nut (310) from the leadscrew when the force is applied to the handle assembly (210).

7. The apparatus (100, 500, 600) of any one of the preceding claims, wherein the handle assembly (210) comprises a moveable handle (211) and a stationary handle (212), wherein the moveable handle (211) is fixedly attached to the cam disc (240), and wherein the stationary handle (212) is fixedly attached to the bottom plate (220) or to a top plate (230).

8. The apparatus (100, 500, 600) of any one of the preceding claims, wherein the underside of the cam disc (240) comprises arched grooves (245) configured to facilitate rotational movement of the cam disc (240), wherein the rotational movement of the cam disc (240) causes the cam (241) to come into contact with and actuate the split nut (310) away from the leadscrew, thereby disengaging the split nut (310) from the leadscrew.

9. The apparatus (100, 500, 600) of any one of the preceding claims, wherein the at least one jaw (251) comprises a tilted surface (256), wherein the tilted surface (256) is configured to interlock with the dispenser.

10. The apparatus (100, 500, 600) of claim 9, wherein the dispenser is interlocked between the upper edge of the tilted surface (256) of the jaw (251) and the bottom surface (255) of the spacer (254).

11. The apparatus (100, 500, 600) of any one of the preceding claims, wherein the cam disc (240) comprises at least one protrusion, wherein the at least one protrusion extends through an opening of the bottom plate (220).

12. The apparatus (100, 500, 600) of any one of the preceding claims, further comprising a spring (246) arranged around the center axle of the cam disc (240), wherein one end of the spring (246) is fixedly attached within a spring housing 247 of the cam disc 240, and another end of the spring (246) is positioned adjacent to the outer edge of the cam disc (240).

13. A liquid handling apparatus (700), comprising at least the apparatus (100, 500, 600) of claim 1, and an actuator configured to actuate the apparatus (100, 500, 600) of claim 1 along a vertical axis via a leadscrew.

14. A method of gripping a dispenser, the method comprising:
- applying a force to a movable handle (211) of an apparatus (100, 500, 600), the apparatus (100, 500, 600) configured so that at least one jaw assembly (250) moves centripetally away from the center of a cam disc (240) upon application of said force; wherein each jaw assembly (250) comprises a jaw (251), a spacer (254), and a jaw slider (253)
- placing a top surface of the dispenser against the underside of each spacer (254) within an area defined by the at least one jaw assembly (250); and
- releasing the force on the movable handle (211), the apparatus (100, 500, 600) configured so that the releasing of the force moves the at least one jaw assembly (250) centripetally towards the center of the cam disc (240), thereby fixing the dispenser in a cavity within the at least one jaw assembly (250), whereby horizontal and vertical movement of the dispenser is prevented.

15. The method of claim 14, wherein the releasing of a force simultaneously engages the split nut (310) to a leadscrew, thereby securing the apparatus (100, 500, 600) to the leadscrew and preventing movement along a vertical axis.
